(19) Europäisches Patentamt
European Patent Office
Office européen des brevets

(11) EP 1 233 944 B1

(12) EUROPÄISCHE PATENTSCHRIFT

(45) Veröffentlichungstag und Bekanntmachung des Hinweises auf die Patenterteilung:
**31.03.2004 Patentblatt 2004/14**

(21) Anmeldenummer: **00987278.9**

(22) Anmeldetag: **21.11.2000**

(51) Int Cl.[7]: **C07D 201/00**

(86) Internationale Anmeldenummer:
**PCT/EP2000/011600**

(87) Internationale Veröffentlichungsnummer:
**WO 2001/038302 (31.05.2001 Gazette 2001/22)**

(54) **KUPPLUNGS-KONDENSATIONS-SYNTHESE VON HETEROCYCLEN**

COUPLING CONDENSATION SYNTHESIS OF HETEROCYCLES

SYNTHESE D'HETEROCYLES PAR CONDENSATION DE COUPLAGE

(84) Benannte Vertragsstaaten:
**AT BE CH CY DE DK ES FI FR GB GR IE IT LI LU MC NL PT SE TR**

(30) Priorität: **21.11.1999 DE 19955634**

(43) Veröffentlichungstag der Anmeldung:
**28.08.2002 Patentblatt 2002/35**

(73) Patentinhaber: **Morphochem AG**
**81379 München (DE)**

(72) Erfinder:
- **MUELLER, Thomas**
  **81379 München (DE)**
- **ANSORGE, Markus**
  **80335 München (DE)**

(74) Vertreter:
**Forstmeyer, Dietmar, Dr. rer. nat., Dipl.-Chem. et al**
**Boeters & Bauer,**
**Bereiteranger 15**
**81541 München (DE)**

(56) Entgegenhaltungen:
- **DAS P ET AL: "Palladium-catalysed synthesis of some biologically active 5,6-disubstituted uracils" BIOORGANIC & MEDICINAL CHEMISTRY LETTERS,GB,OXFORD, Bd. 6, Nr. 20, 22. Oktober 1996 (1996-10-22), Seiten 2477-2480, XP004135862 ISSN: 0960-894X**
- **A. ATTIA ET AL.: "Azachalcones. IV+. Reactions of Azachalcones with O-Phenylenediamine" GAZZETTA CHIMICA ITALIANA, Bd. 112, Nr. 9/10, 25. März 1982 (1982-03-25), Seiten 387-390, XP000985350**
- **A. ATTIA ET AL.: "Azachalcones" PHARMAZIE, Bd. 37, Nr. 8, 30. März 1981 (1981-03-30), Seiten 551-553, XP000986036**
- **S. M. DESENKO ET AL.: "5,7-Triaryl-5,6-Dihydro-4H-Tetrazolo[1,5-b]-1,2,4-Triazepines" CHEMISTRY OF HETEROCYCLIC COMPOUNDS, Bd. 26, 7. Februar 1989 (1989-02-07), Seiten 1278-1280, XP000985757**
- **V. D. ORLOV ET AL.: "2,4-Diaryl-2,3-dihydro-1H-pyrimido[5,6-b] -1,5-diazepines" CHEMISTRY OF HETEROCYCLIC COMPOUNDS, Bd. 27, 27. Juni 1989 (1989-06-27), Seiten 205-210, XP000985756**
- **I. UGI ET AL.: "Multicomponent Reactions in organc chemistry" ENDEAVOUR, Bd. 18, 1994, Seiten 115-122, XP000986032**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 233 944 B1

Printed by Jouve, 75001 PARIS (FR)

## Beschreibung

Die Erfindung betrifft ein Verfahren zur Herstellung von aromatischen oder nichtaromatischen Heterocyclen mit 4 bis 10, vorzugsweise 5 bis 7 Ringgliedern. Diese Heterocyclen finden als Fungizide, Antimikrobiotika, Bakterizide Verwendung.

Im Stand der Technik werden derartige Ringsysteme wie folgt hergestellt.

Durch alkalische Kondensation (Aldolkondensation) eines aromatischen oder heteroaromatischen Aldehyds mit Acetylaromaten oder -heteroaromaten bilden sich 1,3-diaryl- bzw. diheteroarylsubstituierte Enone (Chalcone). Diese Substanzen werden isoliert und gereinigt. In einer zweiten Reaktion werden dann die Chalcone mit bifunktionellen Nucleophilen, wie beispielsweise Hydrazinen, zur Reaktion gebracht. Dabei erhält man die obenerwähnten Ringsysteme.

Dieses obengenannte Verfahren hat den Nachteil, daß es sich hier nicht um ein Eintopfverfahren handelt. Unter einem Eintopfverfahren wird ein insbesondere mehrstufiges Syntheseverfahren verstanden, bei dem eventuelle Zwischenprodukte oder Intermediate nicht aufgearbeitet, d.h. zum Beispiel isoliert, konzentriert oder gereinigt, werden.

Weitere Definitionen für im folgenden verwendete Bezeichnungen:

Unter einem elektronenarmen Aromaten, Heteroaromaten oder Olefin/Alkin bzw. allgemein elektronenarmen System, wird ein System verstanden, dessen $\pi$-Elektronendichte durch negative Induktionseffekte oder negative Mesomerieeffekte (-I-Effekte bzw. -M-Effekte) verringert ist. Eine Aufstellung von Substituenten oder Gruppen, die diese Effekte hervorrufen, findet sich in jedem Standardlehrbuch der organischen Chemie. Als Beispiele seien ohne Beschränkung genannt für-I- Substituenten: OH, Halogene, $NO_2$, ungesättigte Gruppen; für-M- Substituenten: $NO_2$, CN, Aromaten. Diese elektronenziehenden Gruppen (EWG, engl. "electron withdrawing groups") müssen natürlich in Konjugation zur Abgangsgruppe X stehen, d.h. bei Carbocyclen in ortho- oder para-Stellung, um den gewünschten Effekt ausüben zu können.

Unter einer Acceptorgruppe wird eine Gruppe verstanden, die folgende allgemeine Eigenschaften hat: Stabilisierung von negativen Ladungen und Partialladungen durch Delokalisierung über p-Atomorbitale bzw. $\pi$-Molekülorbitale (mesomere Stabilisierung durch -M-Resonanzeffekte, $\pi$-Acceptor) und/oder induktive oder Feldeffekte von elektronegativen Atomen bzw. Molekülteilen (induktive Stabilisierung durch -I-Feldeffekte, $\sigma$-Acceptor) und Kombinationen dieser beiden Effekte (Def. Siehe auch Lehrbücher der Organischen Chemie, z. B. Jerry March, Advanced Organic Chemistry, 4$^{th}$ edition, Wiley-Interscience, New York, Chichester, Brisbane, Toronto, Singapore, S. 17ff, 36).

Als Beispiele seien ohne Beschränkung genannt für Acceptorgruppen: Carbon-, Sulfon-, Phosphon- und Boronsäuren, sowie deren Ester, Amide, Imide, Hydrazide; Cyanogruppe, Ketogruppe, Formylgruppe, Imingruppe, Trifluormethylgruppe, Trialkylammoniumgruppe, Trialkylsilylgruppe,$\eta^6$-PhenylCr(CO)$_{3-}$, $\eta^6$-6-PhenylFe$^+$Cyclopentadienyl)-Komplexe.

Die Aufgabe der vorliegenden Erfindung ist es, ein Eintopfverfahren zur Verfügung zu stellen.

Diese Aufgabe wird durch das erfindungsgemäße Verfahren nach Anspruch 1 gelöst, wobei das Verfahren zur Herstellung von Heterocyclen, dadurch gekennzeichnet ist, daß man die Komponenten:

Die Erfindung betrifft ein Verfahren zur Herstellung von Heterocyclen, dadurch gekennzeichnet, daß man folgende Komponenten:

i) ein Propargylderivat mit der allgemeinen Strukturformel I

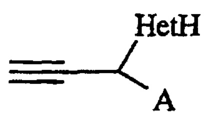

I ,

wobei Het ein gegebenenfalls substituiertes Heteroatom und A ein substituierter oder unsubstituierter Aromat, ein substituierter oder unsubstituierter aromatischer Heterocyclus, ein substituiertes oder unsubstituiertes Vinylaren und/oder ein Derivat davon, ein Olefin, ein Alkin, eine Acceptorgruppe oder ein Nitril ist;

(ii) eine Verbindung mit der allgemeinen Strukturformel II

B— X

II ,

wobei B ein elektronenarmer substituierter oder unsubstituierter Aromat mit oder ohne Acceptorgruppe, ein elektronenarmer substituierter oder unsubstituierter Heteroaromat mit oder ohne Acceptorgruppe, ein elektronenarmes Olefin und/oder Alkin, ein Metallkomplex und X eine Abgangsgruppe ist;

(iii) ein Nucleophil mit der allgemeinen Strukturformel III

$$Y—C_n\text{-}Z$$

III ,

wobei Y und/oder Z unabhängig voneinander eine Aminogruppe, Thiogruppe (Mercaptogruppe), Selenogruppe, Tellurogruppe, Hydroxygruppe (Alkoholgruppe), Imidogruppe, Carbonylgruppe, Thiocarbonylgruppe, Selenocarbonylgruppe, Tellurocarbonylgruppe; C ein substituiertes oder unsubstituiertes C-Atom, eine substituierte oder unsubstituierte oder anellierte CC-Doppelbindung oder -Einfachbindung und n = 0 - 10, vorzugsweise 1 - 5, ist, vorzugsweise in einer Eintopfreaktion durch Cyclokondensation zu 4- bis 10-, vorzugsweise 5- bis 7-gliedrigen, heterocyclischen, aromatischen oder nichtaromatischen Ringsystemen umsetzt.

Weitere vorteilhafte Ausführungsformen sind Gegenstand der Unteransprüche.

Die Substituenten unterliegen keinen besonderen Beschränkungen. Beispiele für geeignete Substituenten (allgemein wird ein Substituent bevorzugt, aber auch zwei, drei oder mehr Substituenten sind erfindungsgemäß möglich) sind in der Beschreibung durchweg, beispielsweise an den Aromaten, Heteroaromaten bzw. aromatischen Heterocyclen oder Vinylarenen, Halogenatome, wie Chlor, Jod, Fluor, Brom, Aromaten wie Phenylgruppen, Alkyl-, Alkoxy-, Amino-, Ester-, Nitril, Nitro-, Aldehyd-, Acetal- oder Sulfongruppen. Die Alkyl- und Alkoxygruppen können geradkettig oder verzweigt sein. Die Kettenlänge kann z.B. 1 bis 25, 1 bis 20, 1 bis 15, 1 bis 10 oder 1 bis 5 Kohlenstoffatome betragen, wie bei Methyl, Ethyl, n-Propyl, iso-Propyl, Butyl-, tert-Butyl; Methyloxy, Ethyloxy, n-Propyloxy, iso-Propyloxy, Butyloxy-, tert-Butyloxy. Die Aminogruppen können primäre, sekundäre oder tertiäre sein. Beispielsweise kann es sich bei den Substituenten am Stickstoffatom um die oben definierten Alkylgruppen handeln. Allgemein können alle Substituenten selber substituiert sein, beispielsweise mit den gleichen Substituenten.

Vorzugsweise wird in dem erfindungsgemäßen Verfahren ferner iv) wenigstens ein geeigneter Metallkatalysator und v) wenigstens eine Aminverbindung und/oder ein Metallsalz, vorzugsweise basische Metallsalze sowie Gemische davon, verwendet.

Erfindungsgemäß vorteilhaft ist es, wenn die Komponenten bei einer Temperatur ≥ 0 °C, bevorzugt ≥ 20 °C, vorzugsweise ≥ 70 °C in einem geeigneten Lösungsmittel umgesetzt werden, wobei geeignete Lösungsmittel aromatische und heteroaromatische Lösungsmittel, aliphatische Ether, Alkohole, Acetonitril, Dimethylformamid, DMSO und Wasser umfassen.

In Komponente i) kann Het z.B. gegebenenfalls substituierte O, S, Se, Te, N, P, As, Sb bedeuten. Vorteilhafterweise ist die Elektronegativität gleich oder größer als die von Kohlenstoff (EN ≥ 2,5). Als Substituenten seien beispielsweise genannt: für N: Tosyl-, Aminooxy-, Acylgruppen, für P, As, Sb: Alkoxy-, Alyloxy-, Phosphorimidgruppen.

Der Substituent bzw. die Gruppe A umfaßt solche Substituenten bzw. Gruppen, die negative Ladungen stabilisieren können, beispielsweise durch Delokalisierung in einem π-Elektronensystem. Besondere Beschränkungen gibt es nicht.

Der Ausdruck " substituierte und konjugierte und carbocylisch und heterocyclisch anellierte" Verbindungen bedeutet, dass die Verbindungen diese Merkmale bzw. Substituenten zugleich oder einzeln aufweisen können, wobei unsubstituierte Verbindungen bevorzugt werden.

Es folgen ohne Beschränkung konkrete Beispiele für die Substituenten A der Komponente i) wobei Alkylgruppen wie vorstehend angegeben definiert sein können:

Aromaten, ausgewählt aus der Gruppe der substituierten und unsubstituierten und konjugierten und carbocylisch und heterocyclisch anellierten Alkyl- und Arylbenzole, Phenole, Arylketone, Arylthioketone, Benzaldehyde, Aniline, Alkyl- und Arylarylether, Benzodioxole, Arylalkyl- und Arylarylthioether, Arylalkylselenoether, Arylarylselenoether, Arylalkyltelluroether, Arylaryltelluroether, Sulfoxybenzole, Sulfonylbenzole, Dialkylarylund Triarylphosphane, Dialkylaryl- und Triarylphosphanoxide, Dialkylaryl- und Triarylarsane, Dialkylaryl- und Triarylarsanoxide, Dialkylaryl- und Triarylstibane, Dialkylaryl- und Triarylstibanoxide, Benzonitrile, Benzoesäureester, Benzoesäurethioester, Benzoesäureselenoester, Benzoesäuretelluroester, Benzamide, Benzthioamide, Benzourethane, Benzoharnstoffe; einschließlich Derivate sowie Gemische davon. Arylreste weisen vorzugsweise 6,10 oder 14 Ring-C-Atome auf. Insbesondere werden Phenole bevorzugt.

Aromatische Heterocyclen bzw. Heteroaromaten, ausgewählt aus der Gruppe der substituierten und unsubstituierten und konjugierten und carbocylisch und heterocyclisch anellierten Thiophene, Selenophene, Tellurophene, Furane, Pyrrole, Indole, Chinoline, Isochinoline, Pyridine, Pyrimidine, Pyrazine, Bipyridine, Bipyrimidine, Bipyrazine, Triazine, Tetrazine, Oxazole, Isooxazole, Thiazole, Imidazole, Triazole, Azepine, Oxazepine, Dioxine, Phenoxazine, Phenothiazine, Porphyrine, Corrine, Phthalocyanine; einschließlich Derivate sowie Gemische davon.

Vinylarene, ausgewählt aus der Gruppe der substituierten und unsubstituierten und konjugierten und carbocylisch und heterocyclisch anellierten Styrole, Indene, Indole, Benzofurane, Benzothiophene, Benzoselenophene; einschließlich Derivate sowie Gemische davon.

Elektronenarme Olefine, ausgewählt aus der Gruppe der substituierten und unsubstituierten und konjugierten und carbocylisch und heterocyclisch anellierten Acrylalkyl- und Arylester, Acrylalkyl- und Arylthioester, Vinylketone, Vinylthioketone, Acroleine, Vinylsulfone, Vinylsulfoxide, Vinylphosphanoxide, Vinylphosphonsäuredialkyl- und diarylester, Acrylnitrile, Acrylamide, Acrylthioamide; einschließlich Derivate sowie Gemische davon. Vorzugsweise weisen die Verbindungen 2 bis 20, besonders bevorzugt 2 bis 10 oder 2 bis 6 C-Atome auf.

Acceptorgruppen, ausgewählt aus der Gruppe der substituierten und unsubstituierten und konjugierten und carbocylisch und heterocyclisch anellierten Trialkylsilylgruppen, Ketogruppen, die z.B. 1 bis 6 C-Atome aufweisen, Formylgruppen, Carbon-, Sulfon-, Phosphon-, Boronsäuren, Benzotriazolgruppen; einschließlich Derivate sowie Gemische davon.

Es folgen ohne Beschränkung konkrete Beispiele für die Substituenten B der Komponente ii):

Elektronenarme Aromaten, ausgewählt aus der Gruppe der substituierten und unsubstituierten und konjugierten und carbocylisch und heterocyclisch anellierten Nitrobenzole, Benzaldehyde, Benzonitrile, Benzoesäureester, Alkylaryl- und Arylarylketone, Alkylarylund Arylarylsulfone, Alkylaryl- und Arylarylsulfoxide, Dialkylaryl- und Triarylphosphanoxide, Dialkylaryl- und Triarylarsanoxide, Dialkylaryl- und Triarylstibanoxide; einschließlich Derivate sowie Gemische davon. Vorzugsweise weisen die Aldehyd-, Ester-, Keto- und/oder Alkylgruppen dieser elektronenarmen Aromaten 1 bis 6 C-Atome auf.

Elektronenarme Heteroaromaten, ausgewählt aus der Gruppe der substituierten und unsubstituierten und konjugierten und carbocylisch und heterocyclisch anellierten Chinoline, Isochinoline, Pyridine, Pyrimidine, Pyrazine, Bipyridine, Bipyrimidine, Bipyrazine, Triazine, Tetrazine, Oxazole, Isooxazole, Thiazole, Imidazole, Triazole; einschließlich Derivate sowie Gemische davon.

Elektronenreiche Heteroaromaten mit mindestens einer Acceptorgruppe, ausgewählt aus der Gruppe der substituierten und unsubstituierten und konjugierten und carbocylisch und heterocyclisch anellierten Thiophene, Selenophene, Tellurophene, Furane, Pyrrole, Indole, Chinoline, Isochinoline, Azepine, Oxazepine, Dioxine, Phenoxazine, Phenothiazine, Porphyrine, Corrine, Phthalocyanine; einschließlich Derivate sowie Gemische davon.

Elektronenarme Olefine, ausgewählt aus der Gruppe der substituierten und unsubstituierten und konjugierten und carbocylisch und heterocyclisch anellierten Acrylalkyl- und Arylester, Acrylalkyl- und Arylthioester, Vinylketone, Vinylthioketone, Acroleine, Vinylsulfone, Vinylsulfoxide, Vinylphosphanoxide, Vinylphosphonsäuredialkyl- und -diarylester, Acrylnitrile, Acrylamide, Acrylthioamide; einschließlich Derivate sowie Gemische davon. Vorzugsweise weisen die Aldehyd-, Ester-, Keto- und/oder Alkylgruppen dieser elektronenarmen Olefine 1 bis 6 C-Atome auf.

Metallkomplexe, ausgewählt aus der Gruppe der substituierten und unsubstituierten und konjugierten und carbocylisch und heterocyclisch anellierten tricarbonylchromkomplexierten Arene und Heteroarene, tricarbonylmangankomplexierten Arensalze, cyclopentadienyleisen- und rutheniumkomplexierten Arensalze, tricarbonyleisenkomplexierten Butadiene, hexacarbonyldicobaltkomplexierten Alkine; einschließlich Derivate sowie Gemische davon.

Es folgen ohne Beschränkung konkrete Beispiele für die Abgangsgruppe X. Halogene, z.B. I, Cl, Br, F, substitutuierte und unsubstituierte Phosphate, Sulfate, Triflate, Nonaflate, Sulfonate, Sulfinate und/oder deren Alkyl- und Arylester; einschließlich Derivate sowie Gemische davon.

Es folgen ohne Beschränkung konkrete Beispiele für die Komponente iii):

Vorteilhaft sind bisfunktionelle oder höher funktionelle Nucleophile, beispielsweise Hydrazine, Hydroxylamine, Harnstoffe, Thioharnstoffe, Amidine und deren Salze, sowie ausgewählt aus der Gruppe der substituierten und unsubstituierten und konjugierten und carbocylisch und heterocyclisch anellierten Phenylendiamine, Aminophenole, Aminothiophenole, Diaminoalkane, Diaminoaryl- und Diaminoheteroarylverbindungen, Aminohydroxyaryl- und Aminohydroxyheteroarylverbindungen, Aminomercaptoaryl- und Aminomercaptoheteroarylverbindungen, Aminoselenoaryl- und Aminoselenoheteroarylverbindungen, β-Aminovinylester, β-Aminovinylketone, β-Aminovinylaldehyde, β-Aminovinylimine, β-Aminovinylsulfone, β-Aminovinyltrifluormethane; einschließlich Derivate sowie Gemische davon. Vorzugsweise weisen die Aldehyd-, Ester-, Keto- und/oder Alkylgruppen dieser Verbindungen 1 bis 6 C-Atome auf.

Es folgen ohne Beschränkung konkrete Beispiele für die Komponente iv):

Als Metallkatalysator eignen sich z.B. Bis(triarylphosphan)palladium(II)halogenide, Bis(triheteroarylphosphan)palladium(II)halogenide, Tetrakis(triarylphosphan)palladium(0), Tetrakis(triheteroarylphosphan)palladium(0), Bis(dibenzylidenaceton)palladium(0) und Triarylphosphane, Palladium(II)halogenide und Triarylphosphane, Palladium(II)-halogenide und Triheteroarylphosphane, Bis(benzonitril)palladium(II)halogenide und Triarylphosphane, Bis(benzonitril)palladium(II)halogenide und Triheteroarylphosphane, Bis(acetonitril)palladium(II)halogenide und Triarylphosphane, Bis(acetonitril)-palladium(II)halogenide und Triheteroarylphosphane; sowie die analogen Nickel- und Platinkomplexe; einschließlich Derivate sowie Gemische davon.

Es folgen ohne Beschränkung konkrete Beispiele für die Komponente v):

Als Aminverbindung eignen sich z.B. aliphatische und/oder aromatische primäre, sekundäre und tertiäre Amine; einschließlich Derivate sowie Gemische davon. Vorzugsweise weisen die sekundären und tertiären Amine Alkylgruppen mit 1 bis 6 C-Atomen auf.

Als Metallsalz eignen sich z.B. Ammonium-, Alkali-, Erdalkali-, Aluminium-, Gallium-, Indium-, Thallium- und Silberacetate, -carbonate und -hydroxide; einschließlich Derivate sowie Gemische davon.

Besonders vorteilhaft sind z.B. Kupfer(I)- und Kupfer(II)halogenide, -acetate, -triflate, -sulfate, carbonate; einschließlich Derivate sowie Gemische davon.

Erfindungsgemäß kann man beispielsweise zum Erhalt der Verbindung 5-[4'-Methyl-2'-(2"-pyridylamino)thiazol-5'yl]-2-(4'-pyridyl)pyrazolin

die Komponente i) mit der Strukturformel

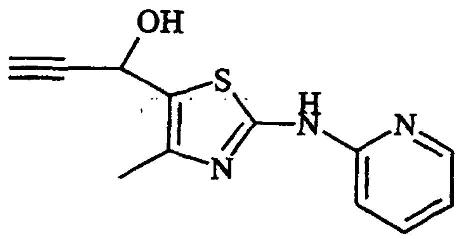

;

die Komponente ü) mit der Strukturformel

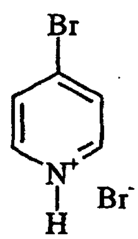

;

und die Komponente iii) mit der Strukturformel

$$H_2N—NH_2 ;$$

cyclokondensieren.

Mit dem erfindungsgemäßen Verfahren lassen sich vorteilhaft aromatische oder nichtaromatische Heterocyclen mit 4 bis 10, vorzugsweise mit 5 bis 7 Ringgliedern, mit fungiziden, antimikrobiotischen und/oder bakteriziden Eigenschaften, umfassend die nachstehenden Verbindungen IV bis VII herstellen.

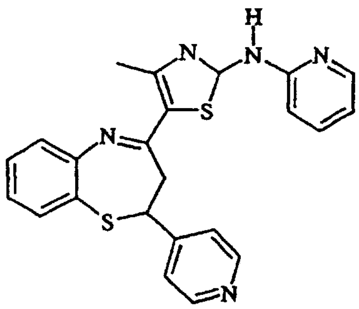

IV

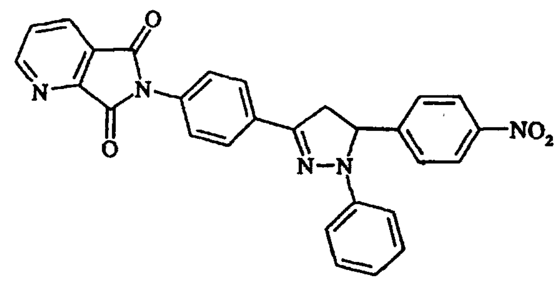

V

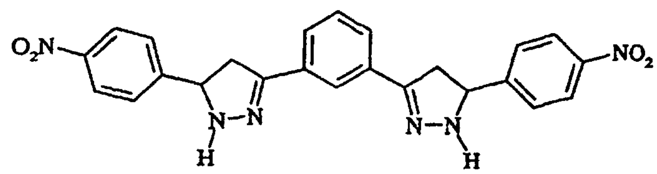

VI

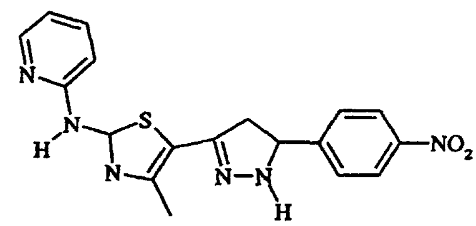

VII

Die nach dem erfindungsgemäßen Verfahren hergestellten Heterocyclen können als Fungizide, Antimikrobiotika und/oder Bakterizide verwendet werden. Es ist klar, daß diese Heterocyclen gleichermaßen für sämtliche dieser Verwendungszwecke geeignet sein können oder auch nur für einzelne. Die Eignung für den jeweiligen Zweck läßt sich durch einfache Routineversuche feststellen.

Möglich ist auch eine fungizide, antimikrobiotische und/oder bakterizide Zusammensetzung, die aus einem oder mehreren nach dem erfindungsgemäßen Verfahren hergestellten Heterocyclen besteht oder, gegebenenfalls in Kombination mit einem für Fungizide, Antimikrobiotika und/oder Bakterizide geeigneten Streckmittel, Hilfsmittel oder Trägermittel, enthält. Es ist klar, daß diese Zusammensetzung eine wirksame Dosis des betreffenden Heterocyclen enthalten muß, welche sich durch einfache Routineversuche feststellen läßt.

Nachstehend wird die Erfindung ohne Beschränkung anhand von konkreten Ausführungsformen und Beispielen erläutert.

Das Interesse an Konsekutivreaktionen nimmt immer stärker zu, da durch effiziente Synthesen in wenigen Schritten, in hoher Chemo-, Regio- und Stereoselektivität, aus einfachen Startmaterialien und in guten Ausbeuten ein Maximum an struktureller Komplexität aufgebaut werden kann. Dabei wird jeweils in der unmittelbar vorangehenden Reaktion die für den Folgeschritt notwendige Funktionalität gebildet. Für die Entwicklung neuer Kaskadenreaktionen, gerade im Hinblick auf Mehrkomponentenreaktionen, ist die *in-situ*-Erzeugung reaktiver funktioneller Gruppen daher eine besondere Herausforderung. Idealerweise laufen dann alle diese Prozesse, gegebenenfalls unter sukzessiver Zugabe der Reagentien, ohne Isolierung der Intermediate im Sinne einer "Eintopfsynthese" ab. Ein Vorbild für solche Konsekutivprozesse sind sowohl Mehrkomponenten-Kondensationen als auch palladiumkatalysierte Kaskadenreaktionen, die sich nicht zuletzt wegen der milden Reaktionsbedingungen und der ausgeprägten Toleranz für funktionelle Gruppen in vielen Fällen als besonders vielfältig gezeigt haben.

Im Laufe von Arbeiten der Erfinder zur Chemie von $AreneCr(CO)_3$-Komplexen mit konjugierten Seitenketten wurde nun gefunden, daß die sonst sehr zuverlässige *Sonogashira-Kupplung* der Chlorarenkomplexe **1** mit 1-Arylprop-2-inolen **2** nicht zu den erwarteten Alkinkupplungsprodukten, den Propargylalkoholen **3**, führt, sondern daß sich in guten Ausbeuten die isomeren arylkomplexierten Chalcone **4** bilden (Schema 1).

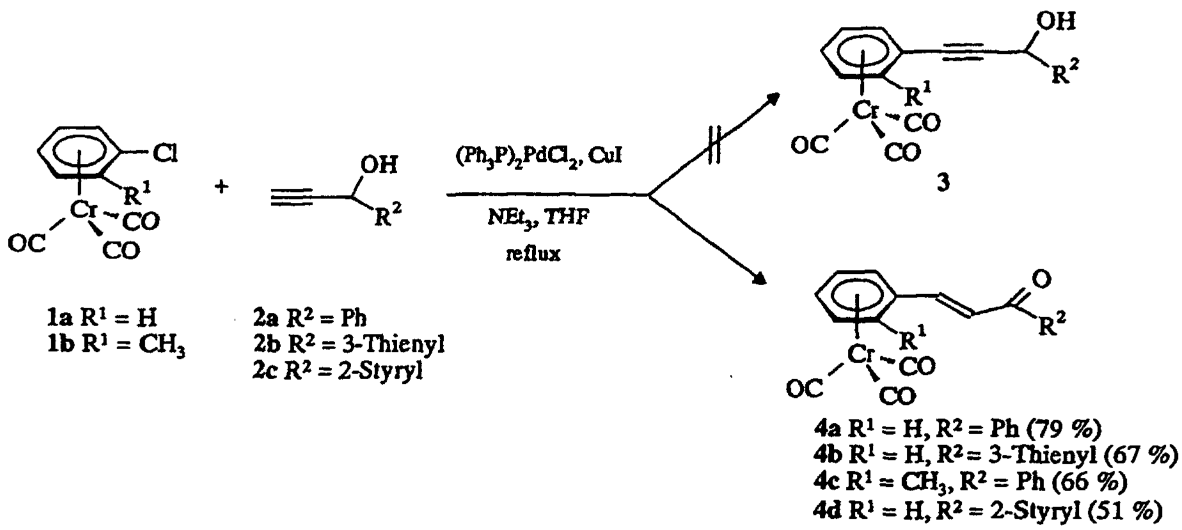


3

**1a** $R^1$ = H
**1b** $R^1$ = $CH_3$

**2a** $R^2$ = Ph
**2b** $R^2$ = 3-Thienyl
**2c** $R^2$ = 2-Styryl

**4a** $R^1$ = H, $R^2$ = Ph (79 %)
**4b** $R^1$ = H, $R^2$ = 3-Thienyl (67 %)
**4c** $R^1$ = $CH_3$, $R^2$ = Ph (66 %)
**4d** $R^1$ = H, $R^2$ = 2-Styryl (51 %)

Schema 1

Mithilfe eines selektiv $^1$H-entkoppelten $^{13}$C-NMR-Experiments konnte die Konstitution des komplexierten Chalcons **4a** zweifelsfrei aufgeklärt und somit eine *Meyer-Schuster-Umlagerung* ausgeschlossen werden. Bemerkenswerterweise entstehen die Chalcone mit ausgezeichneter *trans*-Selektivität ($J^3$ = 16 Hz), was u.a. auf eine thermodynamisch kontrollierte Bildung der Doppelbindung hinweist.

Es wurde gefunden, daß diese Isomerisierung ausschließlich basenkatalysiert abläuft. Denn erhitzt man den komplexierten Diphenylpropargylalkohol **3a** (**$R^1$ = H, $R^2$ = Ph**), das mutmaßliche Intermediat der *Sonogashira-Kupplung* von **1a** mit **2a**, in THF in Gegenwart von Triethylamin, so bildet sich rasch das komplexierte Chalcon **4a** (Schema 2).

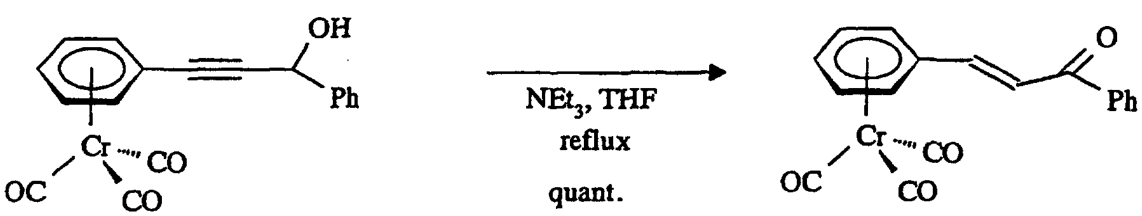


**3a** **4a**

Schema 2

Führt man andererseits die Kupplung des Propargylalkohols **2a** anstelle des Chlorbenzolkomplexes mit Iodbenzol (**5**) durch, das vergleichbar schnell die oxidative Addition am Palladiumkomplex eingeht, so entsteht ausschließlich der Diphenylpropargylalkohol (**6**) (Schema 3). Die elektronische Natur der Halogenarenkomponente spielt offenbar für die nachfolgende Isomerisierung eine Schlüsselrolle. Es wird daher angenommen, daß diese Kupplungs-Isomerisierungs-Sequenz weitaus allgemeiner gültig ist als nur für Heteroaromaten und chromcarbonylkomplexierte Arene.

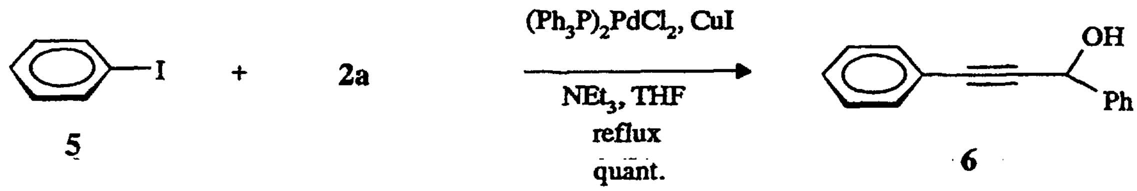


Schema 3

Weist die Halogenkomponente **7** eine elektronenziehende Gruppe (EWG, engl. "electron withdrawing group", z.B. mit -I- oder -M-Effekt) in Konjugation (d.h. bei Carbocyclen eine EWG in ortho- oder para-Stellung) zum Halogenkohlenstoffatom auf, wie z.B. bei acceptorsubstituierten Halogen(hetero)arenen, *Z*-3-Bromacrylsäureester oder 3-Bromcyclohex-2-enon, und ist der Substituent am Propargylzentrum in **2** ein ausgedehntes $\pi$-System, dann entstehen die Enone **8** in guten bis sehr guten Ausbeuten (Schema 4, Tabelle 1).

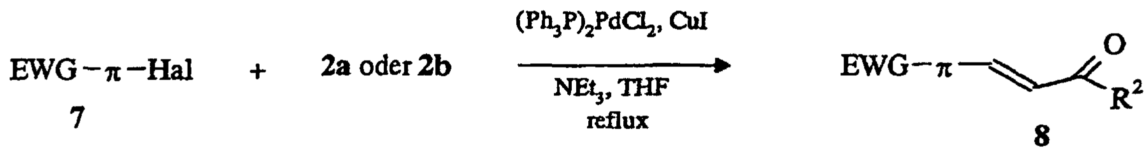


EWG: electron withdrawing group

Schema 4

Aus der Produktanalyse und dem Kontrollexperiment zur basenkatalysierten Isomerisierung von **3a** zu **4a** läßt sich daher der mechanistische Verlauf dieser Kupplungs-Isomerisierungs-Sequenz wie folgt skizzieren (Schema 5).

Die *Sonogashira-Kupplung* des terminalen Arylpropargylalkohols **2** mit einem hinreichend elektronenarmen $sp^2$-hybridisierten Halogenkupplungspartner **7** führt zur Bildung eines acceptorsubstituierten Propargylalkohols **9**. Die Base Triethylamin deprotoniert unter Gleichgewichtsbedingungen am Propargylzentrum, wobei ein resonanzstabilisiertes Propargyl-Allenyl-Anion **10** entsteht, das zum thermodynamisch günstigeren Allen **11** protoniert wird. Die abschließende Allenol-Enon-Tautomerie führt selektiv zur Bildung des *trans*-konfigurierten Enons **8**.

Die milden Reaktionsbedingungen lassen diese Kupplungs-Isomerisierungs-Sequenz als kompetitive Enonsynthese zur Aldolkondensation erscheinen, besonders dann, wenn die entsprechende Aldehydkomponente schwer zugänglich ist (z.B. das korrespondierende 3-Formylcyclohex-2-enon) oder wenn eine alkalisensitive Funktionalität (**7b, 7c, 7d**) ohne aufwendige Schutzgruppenstrategie durch die Reaktion gebracht werden soll. Auch sind Propargylalkohole wesentlich leichter zu handhaben als Vinylketone, die im Sinne einer *Heck-Reaktion* zu Enonen gekuppelt werden können, aber ausgeprägt zur Polymerisation neigen. Interessanterweise führt die Kupplungs-Isomersierungs-Reaktion zu einer vollständigen *Z-E*-Isomerisierung der Doppelbindung des Acrylesterfragmentes (**8e**), was u.a. auch eine thermodynamisch kontrollierte Bildung der Enonfunktionalität (*via* Delokalisierung der intermediär erzeugten negativen Ladung am Propargylzentrum) stützt. *Sonogashira-Kupplungen* hingegen verlaufen stereospezisch unter Erhalt der Alkenkonfiguration. Auf diese Weise lassen sich leicht und stereoselektiv elektronenarme Diene (**8e, 8f**) synthetisieren, die ihrerseits interessante Substrate für Diels-Alder-Reaktionen mit inversem Elektronenbedarf darstellen.

Tabelle 1. Kupplungs-Isomerisierungs-Reaktion.

| Nr. | EWG-π-Hal 7 | Propargylalkohol 2 | Enon 8[a] | Ausb.[%][b] |
|---|---|---|---|---|
| 1 | 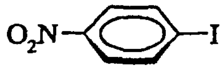 7a | 2a $R^2$ = Ph | 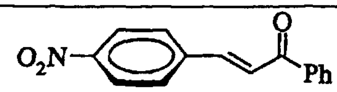 8a | 80 |
| 2 | 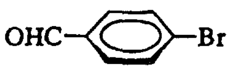 7b | 2a $R^2$ = Ph | 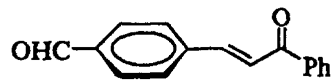 8b | 57 |
| 3 | 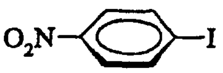 7a | 2b $R^2$ = 3-Thienyl | 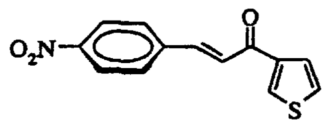 8c | 100 |
| 4 | 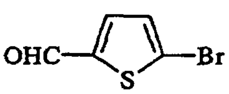 7c | 2a $R^2$ = Ph | 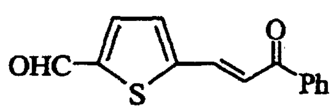 8d | 85 |
| 5 | 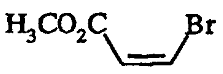 7d | 2b $R^2$ = 3-Thienyl | 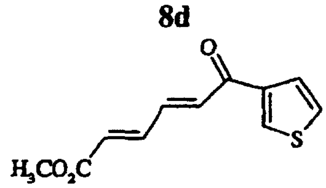 8e | 90 |
| 6 | 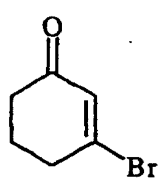 7e | 2b $R^2$ = 3-Thienyl | 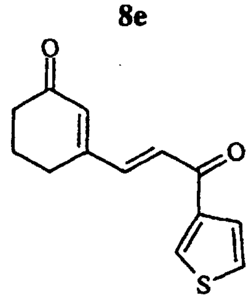 8f | 97 |

[a]Die Verbindungen 8 wurden eindeutig $^1$H- und $^{13}$C-NMR-, IR- und massenspektroskopisch und elementaranalytisch bzw. durch hochaufgelöste Massenspektrometrie charakterisiert. [b]Die Ausbeuten beziehen sich auf isolierte und durch Chromatographie gereinigte Produkte.

Des weiteren ermöglicht diese milde Enonsynthese die Entwicklung neuer Eintopfreaktionen, die unter Ausnutzung der neuentstandenen Enonfunktionalität ablaufen können. Bei den Reaktionsbedingungen ist u.a. nach erfolgter Kupplung der Zusatz von *N*-Methylhydrazin möglich. Denn erst nach der basenkatalysierten Isomerisierung der Propargylalkohole zu den Enonen reagiert das Hydrazin durch Michael-Addition im Sinne einer cyclisierenden Kondensation mit den α,β-ungesättigten Carbonylverbindungen unter Bildung der 2-Pyrazoline **12** (Schema 6, Tabelle 2). Dabei ist es nicht nötig das Enon zuvor zu isolieren.

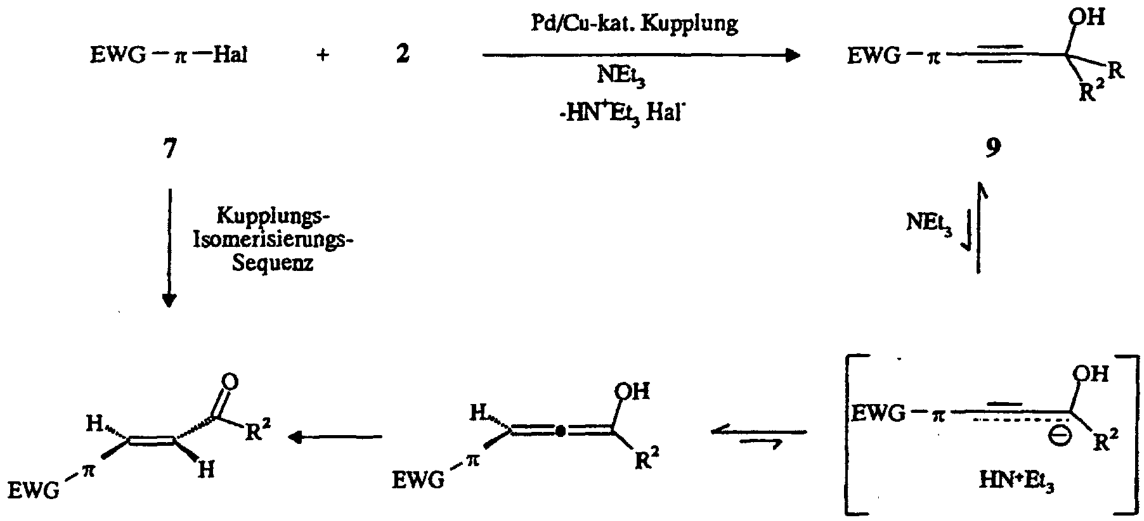


7 9

8 11 10

EWG: electron withdrawing group

Schema 5

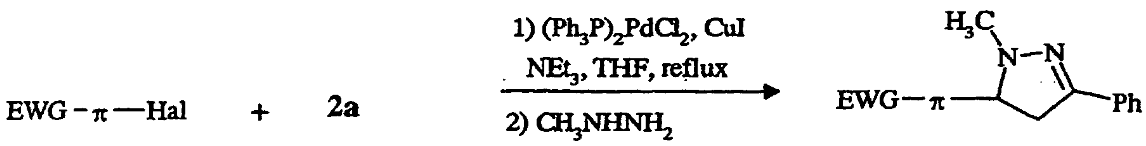


EWG: electron withdrawing group

12

Schema 6

Die erfindungsgemäße Eintopfsynthese von 3,5-disubstituierten 2-Pyrazolinen, einer wichtigen Pharmakophorklasse, erlaubt neue Retrosyntheseschnitte zum Aufbau heterocyclischer Systeme unter Anwendung einer Kupplungs-Isomerisierungs-Sequenz aus einem Arylhalogenid, einem Propargylalkohol und einem Hydrazin (Schema 7). Weitere Heterocyclensynthesen, auch im kombinatorischen Sinne, sind nun zwanglos auf Basis dieser Kupplungs-Isomerisierungs-Sequenz konzipierbar.

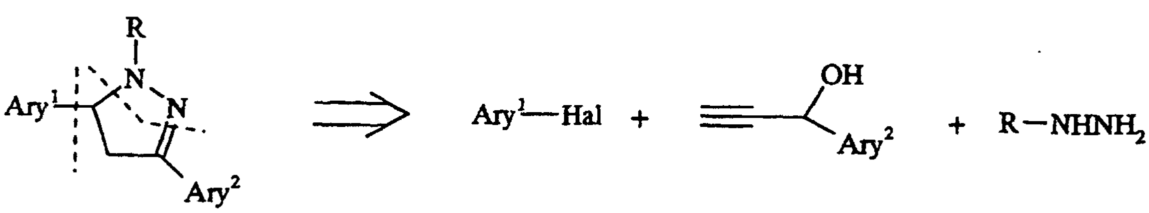

Schema 7

Tabelle 2. Dreikomponenten-Pyrazolin-Synthese

| Nr. | EWG-π-Hal | Pyrazolin 12[a] | Ausb.[%][b] |
|---|---|---|---|
| 1 | 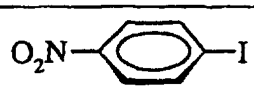 7a | 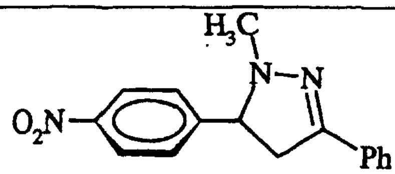 12a | 90 |
| 2 | 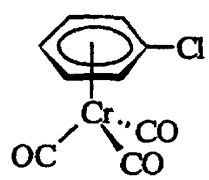 1a | 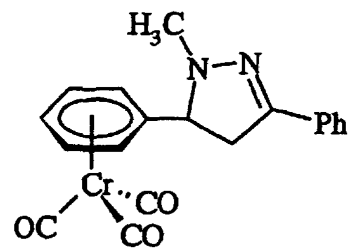 12b | 63 |
| 3 | 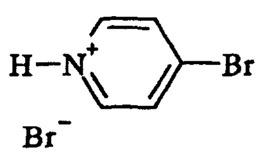 7f | 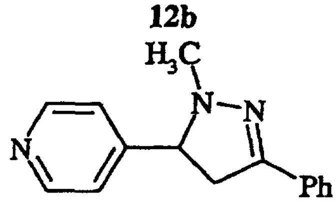 12c | 77 |
| 4 | 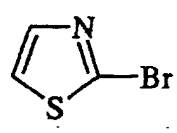 7g | 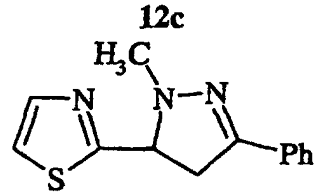 12d | 69 |

[a]Die Verbindungen **12** wurden eindeutig NMR-, IR- und massenspektroskopisch und elementaranalytisch bzw. durch hochaufgelöste Massenspektrometrie charakterisiert. [b]Die Ausbeuten beziehen sich auf isolierte und durch Chromatographie gereinigte Produkte.

## Beispiele

**Enon-Synthese (8a):** Zu einer Lösung von 0.25 g (1.00 mmol) **7a**, 22 mg (0.02 mmol) $Pd(PPh_3)_2Cl_2$ und 2 mg (0.01 mmol) CuI in 10 ml wasserfreiem THF und 5 ml Triethylamin unter Stickstoff tropft man über 30 min eine Lösung von 0.14g (1.05 mmol) **2a** in 10 ml THF zu und erhitzt dann die Reaktionsmischung 10 Stunden unter Rückfluß zum Sieden. Nach dem Abkühlen versetzt man die Mischung mit 30 ml Diethylether und filtriert. Das nach Entfernen der Lösungsmittel im Vakuum erhaltene Rohprodukt wird adsorptiv an Kieselgel (10 cm, Durchmesser 1 cm; Eluent: Diethylether/Pentan) filtriert. Dabei erhält man 0.40 g (80 %) **8a**, Schmp. 161 - 162°C.

**Eintopf-Pyrazolinsynthese (12a):** Zu einer Lösung von 0.25 g (1.00 mmol) **7a**, 22 mg (0.02 mmol) Pd $(PPh_3)_2Cl_2$ und 2 mg (0.01 mmol) CuI in 10 ml wasserfreiem THF und 5 ml Triethylamin unter Stickstoff tropft man während 30 min eine Lösung von 0.14g (1.05 mmol) **2a** in 10 ml THF zu und erhitzt dann die Reaktionsmischung 10 Stunden lang unter Rückfluß zum Sieden. Nach dem Abkühlen versetzt man die Mischung mit 0.15 ml (3.76 mmol) *N*-Methylhydrazin und erhitzt weitere 5 h unter Rückfluß zum Sieden. Das nach Entfernen der Lösungsmittel im Vakuum erhaltene Rohprodukt wird adsorptiv an Kieselgel (10 cm, Durchmesser 1 cm; Eluent: Diethylether/Pentan) filtriert. Dabei erhält man 026 g (92 %) **12a**, Schmp. 118 - 119°C.

**Patentansprüche**

1. Verfahren zur Herstellung von Heterocyclen, **dadurch gekennzeichnet, daß** man folgende Komponenten:

   i) ein Propargylderivat mit der allgemeinen Strukturformel I

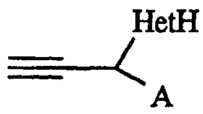

**I** ,

   wobei Het ein gegebenenfalls substituiertes Heteroatom und A ein substituierter oder unsubstituierter Aromat, ein substituierter oder unsubstituierter aromatischer Heterocyclus, ein substituiertes oder unsubstituiertes Vinylaren und/oder ein Derivat davon, ein Olefin, ein Alkin, eine Acceptorgruppe oder ein Nitril ist;

   ii) eine Verbindung mit der allgemeinen Strukturformel II

$$B—X$$

II ,

   wobei B ein elektronenarmer substituierter oder unsubstituierter Aromat mit oder ohne Acceptorgruppe, ein elektronenarmer substituierter oder unsubstituierter Heteroaromat mit oder ohne Acceptorgruppe, ein elektronenarmes Olefin und/oder Alkin, ein Metallkomplex und X eine Abgangsgruppe ist;

   iii) ein Nucleophil mit der allgemeinen Strukturformel III

$$Y—C_n—Z$$

III ,

   wobei Y und/oder Z unabhängig voneinander eine Aminogruppe, Thiogruppe (Mercaptogruppe), Selenogruppe, Tellurogruppe, Hydroxygruppe (Alkoholgruppe), Imidogruppe, Carbonylgruppe, Thiocarbonylgruppe, Selenocarbonylgruppe, Tellurocarbonylgruppe; C ein substituiertes oder unsubstituiertes C-Atom, eine substituierte oder unsubstituierte oder anellierte CC-Doppelbindung oder -Einfachbindung und n = 0 - 10, vorzugsweise 1 - 5, ist, vorzugsweise in einer Eintopfreaktion durch Cyclokondensation zu 4- bis 10-, vorzugsweise 5- bis 7-gliedrigen, heterocyclischen, aromatischen oder nichtaromatischen Ringsystemen umsetzt.

2. Verfahren nach Anspruch 1, wobei ferner

   iv) wenigstens ein geeigneter Metallkatalysator; und

   v) wenigstens eine Aminverbindung und/oder ein Metallsalz, vorzugsweise basische Metallsalze sowie Gemische davon, verwendet wird.

3. Verfahren nach Anspruch 1 oder 2, wobei man die Komponenten bei einer Temperatur ≥ 0 °C, vorzugsweise ≥ 70 °C, in einem geeigneten Lösungsmittel umsetzt, wobei geeignete Lösungsmittel aromatische und heteroaromatische Lösungsmittel, aliphatische Ether, Alkohole, Acetonitril, Dimethylformamid, DMSO und Wasser umfassen.

4. Verfahren nach Anspruch 1, 2 oder 3, wobei der Substituent A der Komponente i) ein Aromat ist, ausgewählt aus der Gruppe der substituierten und unsubstituierten und konjugierten und carbocylisch und heterocyclisch anellierten Alkyl- und Arylbenzole, Phenole, Arylketone, Arylthioketone, Benzaldehyde, Aniline, Alkyl- und Arylarylether,

Benzodioxole, Arylalkyl- und Arylarylthioether, Arylalkylselenoether, Arylarylselenoether, Arylalkyltelluroether, Arylaryltelluroether, Sulfoxybenzole, Sulfonylbenzole, Dialkylaryl- und Triarylphosphane, Dialkylaryl- und Triarylphosphanoxide, Dialkylaryl- und Triarylarsane, Dialkylaryl- und Triarylarsanoxide, Dialkylaryl- und Triarylstibane, Dialkylaryl- und Triarylstibanoxide, Benzonitrile, Benzoesäureester, Benzoesäurethioester, Benzoesäureselenoester, Benzoesäuretelluroester, Benzamide, Benzthioamide, Benzourethane, Benzoharnstoffe; einschließlich Derivate sowie Gemische davon.

**5.** Verfahren nach Anspruch 1, 2 oder 3, wobei der Substituent A der Komponente i) ein aromatischer Heterocyclus ist, ausgewählt aus der Gruppe der substituierten und unsubstituierten und konjugierten und carbocylisch und heterocyclisch anellierten Thiophene, Selenophene, Tellurophene, Furane, Pyrrole, Indole, Chinoline, Isochinoline, Pyridine, Pyrimidine, Pyrazine, Bipyridine, Bipyrimidine, Bipyrazine, Triazine, Tetrazine, Oxazole, Isooxazole, Thiazole, Imidazole, Triazole, Azepine, Oxazepine, Dioxine, Phenoxazine, Phenothiazine, Porphyrine, Corrine, Phthalocyanine; einschließlich Derivate sowie Gemische davon.

**6.** Verfahren nach Anspruch 1, 2 oder 3, wobei der Substituent A der Komponente i) ein Vinylaren ist, ausgewählt aus der Gruppe der substituierten und unsubstituierten und konjugierten und carbocylisch und heterocyclisch anellierten Styrole, Indene, Indole, Benzofurane, Benzothiophene, Benzoselenophene; einschließlich Derivate sowie Gemische davon.

**7.** Verfahren nach Anspruch 1, 2 oder 3, wobei der Substituent A der Komponente i) ein elektronenarmes Olefin ist, ausgewählt aus der Gruppe der substituierten und unsubstituierten und konjugierten und carbocylisch und heterocyclisch anellierten Acrylalkyl- und Arylester, Acrylalkyl- und Arylthioester, Vinylketone, Vinylthioketone, Acroleine, Vinylsulfone, Vinylsulfoxide, Vinylphosphanoxide, Vinylphosphonsäuredialkyl- und -diarylester, Acrylnitrile, Acrylamide, Acrylthioamide; einschließlich Derivate sowie Gemische davon.

**8.** Verfahren nach Anspruch 1, 2 oder 3, wobei der Substituent B der Komponente ii) ein elektronenarmer Aromat ist, ausgewählt aus der Gruppe der substituierten und unsubstituierten und konjugierten und carbocylisch und heterocyclisch anellierten Nitrobenzole, Benzaldehyde, Benzonitrile, Benzoesäureester, Alkylaryl- und Arylarylketone, Alkylaryl- und Arylarylsulfone, Alkylaryl- und Arylarylsulfoxide, Dialkylaryl- und Triarylphosphanoxide, Dialkylaryl- und Triarylarsanoxide, Dialkylarylund Triarylstibanoxide; einschließlich Derivate sowie Gemische davon.

**9.** Verfahren nach Anspruch 1, 2 oder 3, wobei der Substituent B der Komponente ii) ein elektronenarmer Heteroaromat ist, ausgewählt aus der Gruppe der substituierten und unsubstituierten und konjugierten und carbocylisch und heterocyclisch anellierten Chinoline, Isochinoline, Pyridine, Pyrimidine, Pyrazine, Bipyridine, Bipyrimidine, Bipyrazine, Triazine, Tetrazine, Oxazole, Isooxazole, Thiazole, Imidazole, Triazole; einschließlich Derivate sowie Gemische davon.

**10.** Verfahren nach Anspruch 1, 2 oder 3, wobei der Substituent B der Komponente ü) ein elektronenarmer Heteroaromat mit mindestens einer Acceptorgruppe ist, ausgewählt aus der Gruppe der substituierten und unsubstituierten und konjugierten und carbocylisch und heterocyclisch anellierten Thiophene, Selenophene, Tellurophene, Furane, Pyrrole, Indole, Chinoline, Isochinoline, Azepine, Oxazepine, Dioxine, Phenoxazine, Phenothiazine, Porphyrine, Corrine, Phthalocyanine; einschließlich Derivate sowie Gemische davon.

**11.** Verfahren nach Anspruch 1, 2 oder 3, wobei der Substituent B der Komponente ü) ein elektronenarmes Olefin ist, ausgewählt aus der Gruppe der substituierten und unsubstituierten und konjugierten und carbocylisch und heterocyclisch anellierten Acrylalkyl- und Arylester, Acrylalkyl- und Arylthioester, Vinylketone, Vinylthioketone, Acroleine, Vinylsulfone, Vinylsulfoxide, Vinylphosphanoxide, Vinylphosphonsäuredialkyl- und -diarylester, und Acrylnitrile, Acrylamide, Acrylthioamide; einschließlich Derivate sowie Gemische davon.

**12.** Verfahren nach Anspruch 1, 2 oder 3, wobei der Substituent B der Komponente ii) ein Metallkomplex ist, ausgewählt aus der Gruppe der substituierten und unsubstituierten und konjugierten und carbocylisch und heterocyclisch anellierten tricarbonylchromkomplexierten Arene und Heteroarene, tricarbonylmangankomplexierten Arensalze, cyclopentadienyleisen- und rutheniumkomplexierten Arensalze, tricarbonyleisenkomplexierten Butadiene, hexacarbonyldicobaltkomplexierten Alkine; einschließlich Derivate sowie Gemische davon.

**13.** Verfahren nach Anspruch 1, 2 oder 3, wobei X eine Abgangsgruppe ist, ausgewählt aus der Gruppe der Halogene, der substituierten und unsubstituierten Phosphate, Sulfate, Triflate, Nonaflate, Sulfonate, Sulfinate und/oder deren Alkyl- und Arylester; einschließlich Derivate sowie Gemische davon.

**14.** Verfahren nach Anspruch 1, 2 oder 3, wobei das Nucleophil üi) ausgewählt ist aus der Gruppe bestehend aus Hydrazinen, Hydroxylaminen, Harnstoffen, Thioharnstoffen, Amidinen und deren Salze, sowie ausgewählt aus der Gruppe der substituierten und unsubstituierten und konjugierten und carbocylisch und heterocyclisch anellierten Phenylendiamine, Aminophenole, Aminothiophenole, Diaminoalkane, Diaminoaryl- und Diaminoheteroarylverbindungen, Aminohydroxyaryl- und Aminohydroxyheteroarylverbindungen, Aminomercaptoaryl- und Aminomercaptoheteroarylverbindungen, Aminoselenoaryl- und Aminoselenoheteroarylverbindungen, β-Aminovinylester, β-Aminovinylketone, β-Aminovinylaldehyde, β-Aminovinylimine, β-Aminovinylsulfone, β-Aminovinyltrifluormethane; einschließlich Derivate sowie Gemische davon.

**15.** Verfahren nach einem der vorherigen Ansprüche, wobei der Metallkatalysator iv) ausgewählt ist aus der Gruppe der Bis(triarylphosphan)palladium(II)halogenide, Bis(triheteroarylphosphan)palladium(II)halogenide, Tetrakis(triarylphosphan)palladium(0), Tetrakis(triheteroarylphosphan)palladium(0), Bis(dibenzylidenaceton)palladium(0) und Triarylphosphanen, Palladium(II)halogenide und Triarylphosphanen, Palladium(II)halogenide und Tribeteroarylphosphanen, Bis(benzonitril)palladium(II)halogenide und Triarylphosphanen, Bis(benzonitril)palladium(II)halogenide und Triheteroarylphosphanen, Bis(acetonitril)palladium(II)halogenide und Triarylphosphanen, Bis(acetonitril) palladium(II)halogenide und Triheteroarylphosphanen; sowie die analogen Nickel- und Platinkomplexe; einschließlich Derivate sowie Gemische davon.

**16.** Verfahren nach einem der vorherigen Ansprüche, wobei die Aminverbindung v) ausgewählt ist aus der Gruppe der aliphatischen und/oder aromatischen primären, sekundären und tertiären Amine; einschließlich Derivate sowie Gemische davon.

**17.** Verfahren nach einem der vorherigen Ansprüche, wobei das Metallsalz v) ausgewählt ist aus der Gruppe der Ammonium-, Alkali-, Erdalkali-, Aluminium-, Gallium-, Indium-, Thallium- und Silberacetate, -carbonate und -hydroxide; einschließlich Derivate sowie Gemische davon.

**18.** Verfahren nach einem der vorherigen Ansprüche, wobei das Metallsalz v) ausgewählt ist aus der Gruppe der Kupfer(I)- und Kupfer(II)halogenide, -acetate, -triflate, -sulfate, carbonate; einschließlich Derivate sowie Gemische davon.

**19.** Verfahren nach einem der vorherigen Ansprüche, wobei man zum Erhalt der Verbindung 5-[4'-Methyl-2'-(2"-pyridylamino)thiazol-5'yl)-2-(4'-pyridyl)pyrazolin die Komponente i) mit der Strukturformel

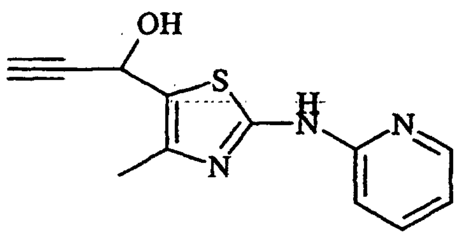

;

die Komponente ii) mit der Strukturformel

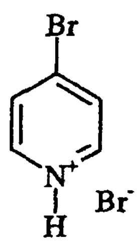

;

und die Komponente iü) mit der Strukturformel

$$H_2N\text{-}NH_2\ ;$$

cyclokondensiert.

**Claims**

1. Process for the preparation of heterocycles, **characterised in that** the following components:

   i) a propargyl derivative of the general structural formula I

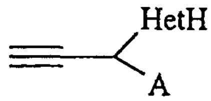

**I** ,

   wherein Het is an optionally substituted hetero atom and A is a substituted or unsubstituted aromatic entity, a substituted or unsubstituted aromatic heterocycle, a substituted or unsubstituted vinyl arene and/or a derivative thereof, an olefin, an alkyne, an acceptor group or a nitrile;

   (ii) a compound of the general structural formula II

B-X

**II,**

   wherein B is an electron-deficient substituted or unsubstituted aromatic entity with or without an acceptor group, an electron-deficient substituted or unsubstituted heteroaromatic entity with or without an acceptor group, an electron-deficient olefin and/or alkyne, a metal complex, and X is a leaving group;

   (iii) a nucleophile of the general structural formula III

$$Y\text{-}C_n\text{-}Z$$

**III,**

   wherein Y and/or Z, each independently of the other, is an amino group, thio group (mercapto group), seleno group, telluro group, hydroxy group (alcohol group), imido group, carbonyl group, thiocarbonyl group, selenocarbonyl group, tellurocarbonyl group; C is a substituted or unsubstituted C atom, a substituted or unsubstituted or annelated CC double bond or single bond and n = 0 - 10, preferably 1 - 5,
   preferably are reacted in a one-pot reaction by cyclocondensation to form 4- to 10-membered, preferably 5- to 7-membered, heterocyclic, aromatic or non-aromatic ring systems.

2. Process according to claim 1, wherein furthermore

   iv) at least one suitable metal catalyst; and

   v) at least one amine compound and/or a metal salt, preferably basic metal salts and mixtures thereof, is used.

3. Process according to claim 1 or 2, wherein the components are reacted at a temperature of ≥ 0°C, preferably ≥ 70°C, in a suitable solvent, suitable solvents including aromatic and heteroaromatic solvents, aliphatic ethers,

alcohols, acetonitrile, dimethylformlamide, DMSO and water.

**4.** Process according to claim 1, 2 or 3, wherein substituent A of component i) is an aromatic entity selected from the group of substituted and unsubstituted and conjugated and carbocyclically and heterocyclically annelated alkyl- and aryl-benzenes, phenols, aryl ketones, aryl thioketones, benzaldehydes, anilines, alkyl- and aryl-aryl ethers, benzodioxoles, arylalkyl and arylaryl thioethers, arylalkyl selenoethers, arylaryl selenoethers, arylalkyl telluroethers, arylaryl telluroethers, sulphoxybenzenes, sulphonyl benzenes, dialkylaryl- and triaryl-phosphanes, dialkylaryl- and triaryl-phosphane oxides, dialkylaryl- and triaryl-arsanes, dialkylaryl- and triaryl-arsane oxides, dialkylaryl- and triaryl-stibanes, dialkylaryl- and triaryl-stibane oxides, benzonitriles, benzoic acid esters, benzoic acid thioesters, benzoic acid selenoesters, benzoic acid telluroesters, benzamides, benzothioamides, benzourethanes and benzoureas; including derivatives and mixtures thereof.

**5.** Process according to claim 1, 2 or 3, wherein substituent A of component i) is an aromatic heterocycle selected from the group of substituted and unsubstituted and conjugated and carbocyclically and heterocyclically annelated thiophenes, selenophenes, tellurophenes, furans, pyrroles, indoles, quinolines, isoquinolines, pyridines, pyrimidines, pyrazines, bipyridines, bipyrimidines, bipyrazines, triazines, tetrazines, oxazoles, isooxazoles, thiazoles, imidazoles, triazoles, azepines, oxazepines, dioxins, phenoxazines, phenothiazines, porphyrins, corrins and phthalocyanines; including derivatives and mixtures thereof.

**6.** Process according to claim 1, 2 or 3, wherein substituent A of component i) is a vinyl arene selected from the group of substituted and unsubstituted and conjugated and carbocyclically and heterocyclically annelated styrenes, indenes, indoles, benzofurans, benzothiophenes and benzoselenophenes; including derivatives and mixtures thereof.

**7.** Process according to claim 1, 2 or 3, wherein substituent A of component i) is an electron-deficient olefin selected from the group of substituted and unsubstituted and conjugated and carbocyclically and heterocyclically annelated acrylic alkyl and aryl esters, acrylic alkyl and aryl thioesters, vinyl ketones, vinyl thioketones, acroleins, vinyl sulphones, vinyl sulphoxides, vinyl phosphane oxides, vinyl phosphonic acid dialkyl and diaryl esters, acrylonitriles, acrylamides and acrylothioamides; including derivatives and mixtures thereof.

**8.** Process according to claim 1, 2 or 3, wherein the substituent B of component ii) is an electron-deficient aromatic entity selected from the group of substituted and unsubstituted and conjugated and carbocyclically and heterocyclically annelated nitrobenzenes, benzaldehydes, benzonitriles, benzoic acid esters, alkylaryl and arylaryl ketones, alkylaryl and arylaryl sulphones, alkylaryl and arylaryl sulphoxides, dialkylaryl and triaryl phosphane oxides, dialkylaryl and triaryl arsane oxides, and dialkylaryl and triaryl stibane oxides; including derivatives and mixtures thereof.

**9.** Process according to claim 1, 2 or 3, wherein substituent B of component ii) is an electron-deficient heteroaromatic entity selected from the group of substituted and unsubstituted and conjugated and carbocyclically and heterocyclically annelated quinolines, isoquinolines, pyridines, pyrimidines, pyrazines, bipyridines, bipyrimidines, bipyrazines, triazines, tetrazines, oxazoles, isooxazoles, thiazoles, imidazoles and triazoles; including derivatives and mixtures thereof.

**10.** Process according to claim 1, 2 or 3, wherein substituent B of component ii) is an electron-deficient heteroaromatic having at least one acceptor group, selected from the group of substituted and unsubstituted and conjugated and carbocyclically and heterocyclically annelated thiophenes, selenophenes, tellurophenes, furans, pyrroles, indoles, quinolines, isoquinolines, azepines, oxazepines, dioxins, phenoxazines, phenothiazines, porphyrins, corrins and phthalocyanines; including derivatives and mixtures thereof.

**11.** Process according to claim 1, 2 or 3, wherein substituent B of component ii) is an electron-deficient olefin selected from the group of substituted and unsubstituted and conjugated and carbocyclically and heterocyclically annelated acrylic alkyl and aryl esters, acrylic alkyl and aryl thioesters, vinyl ketones, vinyl thioketones, acroleins, vinyl sulphones, vinyl sulphoxides, vinyl phosphane oxides, vinyl phosphonic acid dialkyl and diaryl esters, acrylonitriles, acrylamides and acrylothioamides; including derivatives and mixtures thereof.

**12.** Process according to claim 1, 2 or 3, wherein substituent B of component ii) is a metal complex selected from the group of substituted and unsubstituted and conjugated and carbocyclically and heterocyclically annelated tricarbonyl-chromium-complexed arenes and heteroarenes, tricarbonylmanganese-complexed arene salts, cyclopentadienyl-iron- and -ruthenium-complexed arene salts, tricarbonyliron-complexed butadienes and hexacarbonyldicobalt-complexed alkynes; including derivatives and mixtures thereof.

**13.** Process according to claim 1, 2 or 3, wherein X is a leaving group selected from the group of halogens, substituted and unsubstituted phosphates, sulphates, triflates, nonaflates, sulphonates, sulphinates and/or alkyl and aryl esters thereof; including derivatives and mixtures thereof.

**14.** Process according to claim 1, 2 or 3, wherein the nucleophile iii) is selected from the group consisting of hydrazines, hydroxylamines, ureas, thioureas, amidines and salts thereof, and also selected from the group of substituted and unsubstituted and conjugated and carbocyclically and heterocyclically annelated phenylenediamines, aminophenols, aminothiophenols, diaminoalkanes, diaminoaryl and diaminoheteroaryl compounds, aminohydroxyaryl and aminohydroxyheteroaryl compounds, aminomercaptoaryl and aminomercaptoheteroaryl compounds, aminoselenoaryl and aminoselenoheteroaryl compounds, β-aminovinyl esters, β-aminovinyl ketones, β-aminovinyl aldehydes, β-aminovinyl imines, β-aminovinyl sulphones and β-aminovinyl trifluoromethanes; including derivatives and mixtures thereof

**15.** Process according to one of the preceding claims, wherein the metal catalyst iv) is selected from the group of bis(triarylphosphane)palladium(II) halides, bis(triheteroarylphosphane)palladium(II) halides, tetrakis(triarylphosphane)palladium(0), tetrakis(triheteroarylphosphane)palladium(0), bis(dibenzylidenacetone)palladium(0) and triarylphosphanes, palladium(II) halides and triarylphosphanes, palladium(II) halides and triheteroarylphosphanes, bis(benzonitrile)palladium(II) halides and triarylphosphanes, bis(benzonitrile)palladium(II) halides and triheteroarylphosphanes, bis(acetonitrile)palladium(II) halides and triarylphosphanes, and bis(acetonitrile)-palladium(II) halides and triheteroarylphosphanes; and also the analogous nickel and platinum complexes; including derivatives and mixtures thereof.

**16.** Process according to one of the preceding claims, wherein the amine compound v) is selected from the group of aliphatic and/or aromatic primary, secondary and tertiary amines; including derivatives and mixtures thereof.

**17.** Process according to one of the preceding claims, wherein the metal salt v) is selected from the group of acetates, carbonates and hydroxides of ammonium, of alkali metals, of alkaline earth metals, of aluminium, of gallium, of indium, of thallium and of silver; including derivatives and mixtures thereof.

**18.** Process according to one of the preceding claims, wherein the meta! salt v) is selected from the group of halides, acetates, triflates, sulphates and carbonates of copper(I) and of copper(II); including derivatives and mixtures thereof.

**19.** Process according to one of the preceding claims, wherein, to obtain the compound 5-[4'-methyl-2'-(2"-pyridylamino)thiazol-5'-yl]-2-(4'-pyridyl)pyrazoline, there are cyclocondensed component i) of structural formula

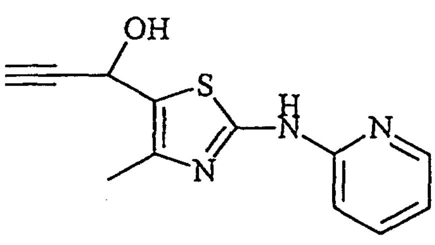

;

component ii) of structural formula

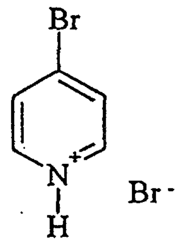

and component iii) of structural formula

$$H_2N—NH_2.$$

**Revendications**

1. Procédé de préparation d'hétérocycles, **caractérisé en ce que** l'on fait réagir les constituants suivants :

   a) un dérivé propargylique de formule structurale générale I

   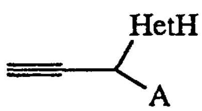

   I

   dans laquelle Het représente un hétéroatome éventuellement substitué, et A représente un hydrocarbure aromatique substitué ou non, un hétérocycle aromatique substitué ou non, un vinyl-arène substitué ou non et/ou un dérivé d'un tel composé, une oléfine, un alcyne ou un nitrile, ou encore un groupe accepteur ;
   b) un composé de formule structurale générale II

   $$B—X \qquad II$$

   dans laquelle B représente un hydrocarbure aromatique substitué ou non, pauvre en électrons et comportant ou non un groupe accepteur, un composé hétéroaromatique substitué ou non, pauvre en électrons et comportant ou non un groupe accepteur, une oléfine et/ou un alcyne pauvre en électrons, ou un complexe métallique, et X représente un groupe partant ;
   c) un composé nucléophile de formule structurale générale III

   $$Y—C_n—Z \qquad III$$

   dans laquelle Y et/ou Z représentent chacun, indépendamment l'un de l'autre, un groupe amino, thiol (mercapto), séléno, telluro, hydroxy (alcool), imido, carbonyle, thiocarbonyle, sélénocarbonyle ou tellurocarbonyle, C représente un atome de carbone substitué ou non, ou une liaison carbone-carbone simple ou double, substituée ou non ou condensée, et n vaut de 0 à 10 et de préférence de 1 à 5,

   par cyclocondensation effectuée de préférence en un seul récipient, pour obtenir des systèmes hétérocycliques, aromatiques ou non, comportant de 4 à 10 et de préférence de 5 à 7 chaînons.

2. Procédé conforme à la revendication 1, dans lequel on utilise en outre :

   d) au moins un catalyseur métallique approprié
   e) et au moins un composé aminé et/ou un sel métallique, de préférence un sel métallique basique, ou un mélange de tels corps.

**3.** Procédé conforme à la revendication 1 ou 2, dans lequel on fait réagir les constituants à une température supérieure ou égale à 0 °C, de préférence supérieure ou égale à 70 °C, dans un solvant approprié, les solvants appropriés englobant les solvants aromatiques ou hétéroaromatiques, les éthers aliphatiques, les alcools, l'acétonitrile, le diméthylformamide, le diméthylsulfoxyde et l'eau.

**4.** Procédé conforme à la revendication 1, 2 ou 3, dans lequel le substituant A du constituant (a) dérive d'un hydrocarbure aromatique choisi dans l'ensemble des alkylbenzènes, arylbenzènes, phénols, arylcétones, arylthiocétones, benzaldéhydes, anilines, alkylaryléthers, arylaryléthers, benzodioxoles, arylalkylthioéthers, arylarylthioéthers, arylalkylsélénoéthers, arylarylsélénoéthers, arylalkyltelluroéthers, arylaryltelluroéthers, benzènesulfoxydes, benzènesulfones, dialkylarylphosphanes, triarylphosphanes, dialkylarylphosphane-oxydes, triarylphosphane-oxydes, dialkylarylarsanes, triarylarsanes, dialkylarylarsane-oxydes, triarylarsane-oxydes, dialkylarylstibanes, triarylstibanes, dialkylarylstibane-oxydes, triarylstibane-oxydes, benzonitriles, esters d'acide benzoïque, thioesters d'acide benzoïque, séléno-esters d'acide benzoïque, telluroesters d'acide benzoïque, benzamides, ben-zothioamides, benzo-uréthanes et benzo-urées, substitués ou non, conjugués ou condensés en carbocycle ou en hétérocycle, y compris leurs dérivés, ainsi que leurs mélanges.

**5.** Procédé conforme à la revendication 1, 2 ou 3, dans lequel le substituant A du constituant (a) dérive d'un hétérocycle aromatique choisi dans l'ensemble des thiophènes, sélénophènes, tellurophènes, furanes, pyrroles, indoles, quinoléines, isoquinoléines, pyridines, pyrimidines, pyrazines, bipyridines, bipyrimidines, bipyrazines, triazines, tétrazines, oxazoles, isoxazoles, thiazoles, imidazoles, triazoles, azépines, oxazépines, dioxines, phénoxazines, phénothiazines, porphyrines, corrines et phtalocyanines, substitués ou non, conjugués ou condensés en carbocycle ou en hétérocycle, y compris leurs dérivés, ainsi que leurs mélanges.

**6.** Procédé conforme à la revendication 1, 2 ou 3, dans lequel le substituant A du constituant (a) dérive d'un vinylarène choisi dans l'ensemble des styrènes, indènes, indoles, benzofuranes, benzothiophènes et benzosélénophènes, substitués ou non, conjugués ou condensés en carbocycle ou en hétérocycle, y compris leurs dérivés, ainsi que leurs mélanges.

**7.** Procédé conforme à la revendication 1, 2 ou 3, dans lequel le substituant A du constituant (a) dérive d'une oléfine pauvre en électrons, choisie dans l'ensemble des acrylates d'alkyle ou d'aryle, thioacrylates d'alkyle ou d'aryle, vinyl-cétones, vinyl-thiocétones, acroléine, vinyl-sulfones, vinyl-sulfoxydes, vinyl-phosphane-oxydes, vinyl-phosphonates de dialkyle ou de diaryle, acrylonitriles, acrylamides et acrylthioamides, substitués ou non, conjugués ou condensés en carbocycle ou en hétérocycle, y compris leurs dérivés, ainsi que leurs mélanges.

**8.** Procédé conforme à la revendication 1, 2 ou 3, dans lequel le substituant B du constituant (b) dérive d'un hydrocarbure aromatique pauvre en électrons, choisi dans l'ensemble des nitrobenzènes, benzaldéhydes, benzonitriles, benzoates, alkylarylcétones, arylarylcétones, alkylarylsulfones, arylarylsulfones, alkylarylsulfoxydes, arylarylsulfoxydes, dialkylarylphosphane-oxydes, triarylphosphane-oxydes, dialkylarylarsane-oxydes, triarylarsane-oxydes, dialkylarylstibane-oxydes, triarylstibane-oxydes, substitués ou non, conjugués ou condensés en carbocycle ou en hétérocycle, y compris leurs dérivés, ainsi que leurs mélanges.

**9.** Procédé conforme à la revendication 1, 2 ou 3, dans lequel le substituant B du constituant (b) dérive d'un composé hétéroaromatique pauvre en électrons, choisi dans l'ensemble des quinoléines, isoquinoléines, pyridines, pyrimidines, pyrazines, bipyridines, bipyrimidines, bipyrazines, triazines, tétrazines, oxazoles, isoxazoles, thiazoles, imidazoles et triazoles, substitués ou non, conjugués ou condensés en carbocycle ou en hétérocycle, y compris leurs dérivés, ainsi que leurs mélanges.

**10.** Procédé conforme à la revendication 1, 2 ou 3, dans lequel le substituant B du constituant (b) dérive d'un composé hétéroaromatique pauvre en électrons et comportant au moins un groupe accepteur, choisi dans l'ensemble des thiophènes, sélénophènes, tellurophènes, furanes, pyrroles, indoles, quinoléines, isoquinoléines, azépines, oxazépines, dioxines, phénoxazines, phénothiazines, porphyrines, corrines et phtalocyanines, substitués ou non, conjugués ou condensés en carbocycle ou en hétérocycle, y compris leurs dérivés, ainsi que leurs mélanges.

**11.** Procédé conforme à la revendication 1, 2 ou 3, dans lequel le substituant B du constituant (b) dérive d'une oléfine pauvre en électrons, choisie dans l'ensemble des acrylates d'alkyle ou d'aryle, thioacrylates d'alkyle ou d'aryle, vinyl-cétones, vinyl-thiocétones, acroléines, vinyl-sulfones, vinyl-sulfoxydes, vinyl-phosphane-oxydes, vinyl-phosphonates de dialkyle ou de diaryle, acrylonitriles, acrylamides et acrylthioamides, substitués ou non, conjugués ou condensés en carbocycle ou en hétérocycle, y compris leurs dérivés, ainsi que leurs mélanges.

**12.** Procédé conforme à la revendication 1, 2 ou 3, dans lequel le substituant B du constituant (b) dérive d'un complexe métallique choisi dans l'ensemble des complexes tricarbonyl-chrome d'hydrocarbures aromatiques ou hétéroaromatiques, sels de complexes tricarbonyl-manganèse d'hydrocarbures aromatiques, sels de complexes cyclopentadiényl-fer ou cyclopentadiényl-ruthénium d'hydrocarbures aromatiques, complexes tricarbonyl-fer de butadiène et complexes hexacarbonyl-dicobalt d'alcynes, substitués ou non, conjugués ou condensés en carbocycle ou en hétérocycle, y compris leurs dérivés, ainsi que leurs mélanges.

**13.** Procédé conforme à la revendication 1, 2 ou 3, dans lequel X représente un groupe partant choisi parmi les atomes d'halogène, les groupes phosphate, sulfate, triflate, nonaflate, sulfonate, sulfinate, et/ou leurs esters d'alkyle ou d'aryle, substitués ou non, y compris leurs dérivés, ainsi que leurs mélanges.

**14.** Procédé conforme à la revendication 1, 2 ou 3, dans lequel le composé nucléophile (c) est choisi dans l'ensemble constitué par les hydrazines, hydroxylamines, urées, thiourées, amidines et leurs sels, ou dans l'ensemble des phénylènediamines, aminophénols, aminothiophénols, diaminoalcanes, composés diaminoaryliques ou diaminohétéroaryliques, composés aminohydroxyaryliques ou aminohydroxyhétéroaryliques, composés aminomercaptoaryliques ou aminomercaptohétéroaryliques, composés aminosélénoaryliques ou aminosélénohétéroaryliques, β-aminovinylesters, β-aminovinylcétones, β-aminovinylaldéhydes, β-aminovinylimines, β-aminovinylsulfones et β-aminovinyltrifluorométhanes, substitués ou non, conjugués ou condensés en carbocycle ou en hétérocycle, y compris leurs dérivés, ainsi que leurs mélanges.

**15.** Procédé conforme à l'une des revendications précédentes, dans lequel le catalyseur métallique (d) est choisi dans l'ensemble des halogénures de bis(triarylphosphane)palladium(II), halogénures de bis(trihétéroarylphosphane) palladium(II), tétrakis(triarylphosphane)palladium(0), tétrakis(trihétéroarylphosphane)palladium(0), bis(dibenzylidène-acétone)palladium(0) et triarylphosphanes, halogénures de palladium(II) et triarylphosphanes, halogénures de palladium(II) et trihétéroarylphosphanes, halogénures de bis(benzonitrile)palladium(II) et triarylphosphanes, halogénures de bis(benzonitrile)palladium(II) et trihétéroarylphosphanes, halogénures de bis(acétonitrile)palladium(II) et triarylphosphanes, halogénures de bis(acétonitrile)palladium(II) et trihétéroarylphosphanes, ainsi que les complexes analogues de nickel ou de platine, y compris leurs dérivés, ainsi que leurs mélanges.

**16.** Procédé conforme à l'une des revendications précédentes, dans lequel le composé aminé (e) est choisi dans l'ensemble des amines primaires, secondaires ou tertiaires aliphatiques et/ou aromatiques, y compris leurs dérivés, ainsi que leurs mélanges.

**17.** Procédé conforme à l'une des revendications précédentes, dans lequel le sel métallique (e) est choisi dans l'ensemble des acétates, carbonates et hydroxydes d'ammonium, des métaux alcalins ou alcalino-terreux, d'aluminium, de gallium, d'indium, de thallium ou d'argent, y compris leurs dérivés, ainsi que leurs mélanges.

**18.** Procédé conforme à l'une des revendications précédentes, dans lequel le sel métallique (e) est choisi dans l'ensemble des halogénures, acétates, triflates, sulfates et carbonates de cuivre(I) ou de cuivre (II), y compris leurs dérivés, ainsi que leurs mélanges.

**19.** Procédé conforme à l'une des revendications précédentes, dans lequel, pour obtenir le composé 5-[4'-méthyl-2'-(2"-pyridylamino)thiazol-5'-yl]-2-(4'-pyridyl)pyrazoline, on effectue la cyclocondensation du composant (a) de formule structurale

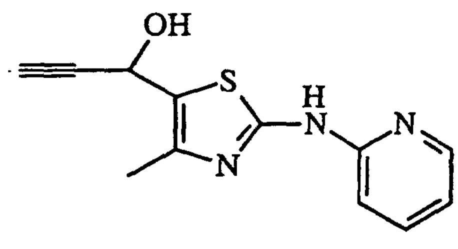

du composant (b) de formule structurale

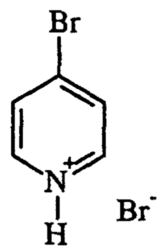

et du composant (c) de formule structurale

$$H_2N-NH_2.$$